Europäisches Patentamt

(19) European Patent Office

Office européen des brevets

(11) Publication number: **0 187 525**
**B1**

(12) **EUROPEAN PATENT SPECIFICATION**

(45) Date of publication of patent specification: **07.11.90**

(21) Application number: **85309455.5**

(22) Date of filing: **23.12.85**

(51) Int. Cl.⁵: **C 12 P 13/06,** C 12 P 17/14,
C 12 P 41/00

(54) Process for producing L-serine.

(30) Priority: **27.12.84 JP 281339/84**
**08.05.85 JP 96895/85**
**08.05.85 JP 96896/85**
**17.07.85 JP 157153/85**

(43) Date of publication of application:
**16.07.86 Bulletin 86/29**

(45) Publication of the grant of the patent:
**07.11.90 Bulletin 90/45**

(84) Designated Contracting States:
**DE FR GB**

(56) References cited:
**PATENT ABSTRACTS OF JAPAN, vol. 1, no. 26,
26th March 1977, page 1084 C 76; & JP-A-51 136
619 (AJINOMOTO K.K.) 26-11-1976**

**PATENT ABSTRACTS OF JAPAN, vol. 1, no. 111,
26th September 1977, page 2575 C 77; &
JP-A-52 72 883 (AJINOMOTO K.K.) 17-06-1977**

(73) Proprietor: **AJINOMOTO CO., INC.**
**5-8, Kyobashi 1-chome, Chuo-ku
Tokyo 104 (JP)**

(72) Inventor: **Majima, Eiji
No. 2-89, Shinmei-cho Saiwai-ku
Kawasaki-shi Kanagawa-ken (JP)**
Inventor: **Izawa, Kunisuke
No. 204-21, Mamedo-cho Kohoku-ku
Yokohama-shi Kanagawa-ken (JP)**
Inventor: **Takino, Hiroaki
No. 2-20-8, Kannn Kawasaki-ku
Kawasaki-shi Kanagawa-ken (JP)**
Inventor: **Yokozeki, Kenzo
No. 1523-4, Shinsaku Takatsu-ku
Kawasaki-shi Kanagawa-ken (JP)**
Inventor: **Kubota, Koji
No. 6-4-6, Nagao Tama-ku
Kawasaki-shi Kanagawa-ken (JP)**

(74) Representative: **Armitage, Ian Michael et al
MEWBURN ELLIS & CO. 2/3 Cursitor Street
London EC4A 1BQ (GB)**

**EP 0 187 525 B1**

(56) References cited:
CHEMICAL ABSTRACTS, vol. 105, no. 25,
December 1986, page 624, abstract no. 224600v,
Columbus, Ohio, US; & JP-A-61 139 397
(SHOWA DENKO K.K.) 26-06-1986

CHEMICAL ABSTRACTS, vol. 103, no. 5, 5th
August 1985, page 539, abstract no. 37736r,
Columbus, Ohio, US; & JP-A-60 34 937 (SHOWA
DENKO K.K.) 22-02-1985

**Description**

The present invention relates to a process for biochemically producing L-serine from 2-oxo-oxazolidine-4-carboxylic acid or a salt thereof (hereinafter abbreviated as OOC).

L-serine is one of the amino acids which constitute proteins and is important as a medical or foodstuff additive or raw material for cosmetics.

L-serine is widely distributed in the natural world as a component of proteins. Thus, L-serine has been produced by hydrolyzing silk yarn, flock, sericin, human hair, swine hair, etc. containing relatively large amounts of L-serine, separating liberated L-serine from other amino acids and purifying it. However, the yield is low in this process. In addition, the process is not always advantageous because it may not be economic owing to a restriction in supply of raw materials.

Some processes for chemical synthesis of L-serine are also known: however, the product is the DL-form which is optically inactive. To separate L-serine from the product, complicated optical resolution must be used which cannot always be applied to the industrial production of L-serine.

As a process for producing L-serine by fermentation there is known a process for producing it from glycine using micoorganisms belonging to e.g. the genus *Corynebacterium,* or the genus *Pseudomonas.* However, the process is not advantageous from the viewpoint of yield, economical considerations, etc.

On the other hand, as an enzymic process for producing L-serine, there is a process utilizing serine hydroxymethyl transferase derived from animals and microorganisms. However, in this process expensive tetrahydrofolate must be used, in addition to glycine and formaldehyde. Further, the process is disadvantageous in that the yield is poor.

The present invention provides an efficient process for producing L-serine by reacting a microorganism or cell product thereof capable of producing L-serine from L-OOC with a starting material comprising L-OOC to produce L-serine. Optionally in addition OOC can be reacted with a microorganism or cell product thereof capable of racemizing OOC, to produce DL-OOC which can provide a source of L-OOC for conversion to L-serine.

Examples of microorganisms capable of racemizing OOC which can be used in the present invention include those shown in the following Table A:

TABLE A

| Agrobacterium radiobacter | AJ 2782 |
| | ATCC 6466 |
| Alcaligenes marchallii | AJ 2147 |
| | ATCC 21030 |
| Arthrobacter citreus | AJ 1423 |
| | ATCC 11624 |
| Bacillus licheniformis | AJ 3290 |
| | ATCC 21417 |
| Beijerinchkia indica | AJ 2821 |
| | ATCC 9037 |
| Brevibacterium ammoniagenes | AJ 1443 |
| | ATCC 6871 |
| Corynebacterium acetoacidphilum | AJ 1550 |
| | ATCC 13870 |
| Escherichia coli | AJ 2621 |
| | ATCC 13070 |
| Mycobacterium ammoniaphilum | AJ 1997 |
| | ATCC 15354 |
| Micrococcus flavus | AJ 1021 |
| | ATCC 400 |
| Pseudomonas oleovorans | AJ 2058 |
| | ATCC 8062 |
| Saricina lutea | AJ 1217 |
| | ATCC 272 |
| Serratia marcescens | AJ 2686 |
| | ATCC 14225 |

**EP 0 187 525 B1**

Bacteria other than those described above may be used in the present invention, as long as they are microorganisms capable of racemizing OOC.

Examples of the microorganisms capable of producing L-serine by hydrolysis of OOC which can be used in the present invention include those shown in the following Table B:

TABLE B

| | |
|---|---|
| Alcaligenes faecalis | AJ 2541 |
| | FERM-P 8030, FERM-BP 940 |
| Arthrobacter grobiformis | AJ 1422 |
| | ATCC 8010 |
| Bacillus subtilis | AJ 1992 |
| | ATCC 13952 |
| Cellulomonas flavigena | AJ 1568 |
| | ATCC 491 |
| Corynebacterium hydrocarboclastus | FERM-P 1097 |
| Flavobacterium aquatile | AJ 2135 |
| | ATCC 8375 |
| Jensenia canicruria | AJ 3147 |
| | ATCC 11048 |
| Mycobacterium ammoniaphilum | AJ 1997 |
| | ATCC 15354 |
| Micrococcus roseus | AJ 1006 |
| | ATCC 9815 |
| Rhodococcus erythropolis | AJ 9126 |
| | ATCC 4277 |
| Pseudomonas testosteroni | AJ 2270 |
| | ATCC 17409 |
| Pseudomonas acidovorans | AJ 3117 |
| | ATCC 15668 |
| Candida zeylanoides | AJ 4677 |
| | IFO 0719 |
| Citeromyces matritensis | AJ 4287 |
| | CBS 2764 |
| Cryptococcus laurentii | AJ 5225 |
| | IFO 0609 |
| Debaryomyces hansenii | AJ 4179 |
| | IFO 0023 |
| Endomycopsis oventensis | AJ 5062 |
| | CBS 2508 |
| Geotricum fragrans | AJ 14298 |
| | CBS 15225 |
| Hansenula californica | AJ 5573 |
| | IFO 0800 |
| Kluyveromyces marxianus | AJ 4074 |
| | IFO 0219 |

4

TABLE B (continued)

| | |
|---|---|
| Nadosonia falvescens | AJ 5332 |
| | IFO 0666 |
| Rhodotorula marina | AJ 5014 |
| | IFO 0879 |
| Torulopsis famata | AJ 4342 |
| | IFO 0623 |
| Trichosporon fermantans | AJ 5152 |
| | IFO 1199 |
| Wickerhamia fluorescens | AJ 4285 |
| | IFO 1116 |
| Achromobacter viscosus | ATCC 12448 |
| Aeromonas salmonicida | ATCC 14174 |
| Agrobacterium radiobacter | ATCC 6466 |
| Azotobacter vinelandii | ATCC 9046 |
| Brevibacterium pusillum | ATCC 19096 |
| Escherichia coli | ATCC 13071 |
| Klebsiella penumonia | ATCC 8329 |
| Kluyvera non-citrophila | FERM-P 3150 |
| Kurthina zophii | ATCC 6900 |
| Mycoplana dimorpha | ATCC 4279 |
| Proteus rettgeri | FERM-P 8196, FERM-BP 941 |
| | AJ 2770 |
| Salmonella schottmuelleri | ATCC 8759 |
| Serratia marcescens | ATCC 14225 |
| Streptomyces humifer | FERM-P 2347 |
| Vibrio tyrogenes | ATCC 7085 |
| Xantomonas canpestris | ATCC 7381 |
| Pichia membranaefaciens | IFO 0460 |
| Saccharomyces fermantati | IFO 0422 |
| Tremella brasiliensis | IFO 9289 |
| Alternaria cucumerina | IFO 7417 |
| Curvularia geniculata | ATCC 6671 |
| Fusarium nivale | ATCC 42308 |
| Helminthosporium gramineum | ATCC 6695 |
| Phoma destructiva | ATCC 24636 |

TABLE B (continued)

| | |
|---|---|
| Sclerotium bataticola | ATCC 12265 |
| Cochliobolus miyabeanus | IFO 5844 |
| Mucor circinelloides | ATCC 8770 |
| Aspergillus repens | ATCC 5817 |
| Penicillium decumbens | ATCC 10436 |
| Gromerella cingulata | ATCC 11326 |
| Septoria glycines | ATCC 38699 |
| Pseudoplea trifolii | IFO 7252 |
| Stemphylium astragali | IFO 7244 |
| Diplodia natalensis | ATCC 34643 |
| Stachylidium bicolor | ATCC 12672 |
| Eupenicillium alutaceum | ATCC 18542 |
| Anixiella reticulata | ATCC 34511 |
| Arachniotus flavoluteus | ATCC 18430 |
| Byssochlamys fulva | ATCC 10099 |
| Coniochaeta tetraspora | ATCC 22275 |
| Gelasinospora longispora | ATCC 18493 |
| Gymnoascus umbrinus | IFO 8358 |
| Microascus cinereus | ATCC 16594 |
| Microthecium retisporum | ATCC 22184 |
| Sordaria humana | ATCC 22796 |
| Sporormiella isomera | ATCC 24341 |
| Toxotrichum cancellatum | ATCC 15316 |
| Melanospora zamiae | ATCC 12340 |

*Alcaligenes faecalis* FERM-P 8030 was originally deposited on 24 December 1984 and *Proteus rettgeri* FERM-P 8196 was originally deposited on 25 April 1985 at the Fermentation Research Institute, Ministry of International Sciences and Technology, Ministry of International Trade and Industry (FRI), 1—3, Higashi 1-Chome, Yatabe-machi, Tsukuba-gun, Ibaragi-ken 305, Japan, and were accorded the FERM-P number indicated above. The microorganisms deposited were then converted into deposits under the Budapest Treaty on 24 November 1985, and were accorded the corresponding FERM-BP numbers.

Microorganisms other than the bacteria described above may be used in the present invention, as long as they are microorganisms capable of producing L-serine through decomposition of OOC.

As media for culturing microorganisms capable of racemizing OOC and microorganisms capable of producing L-serine by hydrolyzing OOC as described above, ordinary nutrient media may be appropriately used. As carbon sources, there may be used, for example, sugars such as glucose, sucrose, glycerol, molasses, organic acids such as fumaric acid, acetic acid, and alcohols such as ethanol and methanol. As nitrogen sources, there may be used for example ammonium sulfate and ammonium chloride. As organic nutrient sources, there may be used for example yeast extract, peptone, meat extract and corn steep liquor.

6

As inorganic ions, there may be used for example ions of magnesium, iron, manganese, potassium, sodium and phosphoric acid. As vitamins, there may be used for example pyridoxine and pyridoxal phosphate. Incubation may be carried out in a conventional manner. For example, the pH of the medium is rendered 6 to 9 and the bacterium is aerobically cultured at 20 to 40°C for 1 to 3 days. Upon incubation, a culture or cell product having a high capability of hydrolysis or racemization may be obtained sometimes by incorporating a small quantity of OOC in the medium.

Cell products which can be used in the present invention are products of the cells which retain the activity of hydrolysing or racemizing OOC. Examples of such cell products include a culture solution per se, a solution obtained by separating cells from the culture solution, separated cells, decomposition products of the separated cells and purified decomposition products; in particular, crude or purified enzyme products from the cells. These cell products may be used as they are, or may be subjected to treatments such as freeze drying, drying with acetone or immobilization.

The concentration of substrate in the enzymic reaction may vary according to whether it is a batch process or continuous process, but is generally from 0.1 to 30%, preferably 0.5 to 10%, in an aqueous medium, in the batch process; whereas in the continuous system, it is preferred that the concentration be somewhat lower than the above ranges.

The reaction is carried out generally in an aqueous medium, at 15 to 60°C, preferably at about 30 to about 40°C, at pH of 4 to 10, preferably about 7. The reaction time varies depending upon the means used for settling, stirring, flowing, etc. and the mode of the titer of enzyme standard. However, in the batch system, the reaction time is generally about 10 minutes to about 72 hours.

Where cells of the above-described microorganisms are brought into contact with OOC while culturing the cells in an aqueous medium, the aqueous medium contains OOC and nutrient sources required for growth of the microorganisms such as carbon sources, nitrogen sources, inorganic ions, etc. In addition, the incorporation of organic trace nutrients such as vitamins, amino acids, etc. often give desired results.

As carbon sources, carbohydrates such as glucose or sucrose, organic acids such as acetic acid, alcohols, and the like may be appropriately used. As nitrogen sources, ammonia gas, aqueous ammonia, ammonium salts and the like may be used. As inorganic ions, magensium ions, phosphate ions, potassium ions, iron ions and the like may be appropriately used as required.

Incubation is suitably carried out while controlling the conditions within appropriate ranges at pH of 4 to 8 and temperatures of 25 to 40°C under aerobic conditions.

On the other hand, where a culture solution of the microorganism is reacted as it is with OOC, or the cultured cells or cell products are reacted by bringing them into contact with OOC; OOC and the culture solution, or an aqueous medium in which the cultured cells or cell products are dissolved or suspended may be allowed to stand or stirred for a while while controlling the temperature to a suitable range between 15 and 60°C and keeping the pH at 4 to 10. Thus after a suitable period, e.g. in the range 10 minutes to 72 hours, large quantities of racemic compounds or hydrolyzed products of OOC are produced and accumulated in the aqueous medium.

Quantitative analysis of the D-form and the L-form by liquid chromatography using a resin for optical resolution was used to determine whether or not OOC was racemized by the enzymic reaction in the present invention. Further, by means for example of NMR spectrum data, X-ray diffraction pattern, liquid chromatography, quantitative bioassay data and specific rotary power, on serine crystals obtained in the examples later described, it was determined whether or not L-serine was produced by hydrolysis of OOC by the enzymic reaction.

## Example 1

In a 500 ml volume flask was charged 50 ml of medium (pH 7.0) containing 2% of glycerol, 0.5% of yeast extract, 0.5% of peptone, 0.25% of NaCl, 0.2% of DL-OOC and 4.0% of calcium carbonate (separately sterilized) followed by sterilization at 120°C for 15 minutes. A microorganism shown in Table 1 cultured at 30°C for 24 hours in bouillon-agar medium was inoculated on the medium. After culturing at 30°C for 24 hours, the cells were centrifuged, washed and collected. The cells were added to acetate buffer (0.1 M at the end; terminal pH, 4.0) or phosphate buffer (0.1 M at the end; terminal pH, 7.0) or tris buffer (1.0 M at the end, terminal pH, 8.5) containing 1% of D-OOC in a 5% concentration calculated as viral cells. The mixture was allowed to stand at 30°C for 24 hours for the reaction to proceed. After completion of the reaction, L-OOC produced was quantitatively determined by liquid chromatography using a resin for optical resolution. The results are shown in Table 1.

TABLE 1
Amount of L-OOC Produced by Various Microorganisms at pH of 4.0, 7.9 or 8.5

| Microorganism | Amount of L-OOC Produced [mg/dl] | | |
|---|---|---|---|
| | pH 4.0 | pH 7.0 | pH 8.5 |
| Agrobacterium radiobacter ATCC 6466 | 0 | 45 | 171 |
| Alcaligenes marchallii ATCC 21030 | 18 | 128 | 264 |
| Arthrobacter citreus ATCC 11624 | 16 | 25 | 85 |
| Bacillus licheniformis ATCC 21417 | 43 | 479 | 362 |
| Beijerinchkia indica ATCC 9037 | 0 | 158 | 341 |
| Brevibacterium ammoniagenes ATCC 6871 | 25 | 417 | 477 |
| Corynebacterium acetoacidphilum ATCC 13870 | 29 | 262 | 265 |
| Escherichia coli ATCC 13070 | 41 | 68 | 0 |
| Mycobacterium ammoniaphilum ATCC 15354 | 0 | 174 | 314 |
| Micrococcus flavus ATCC 400 | 0 | 245 | 315 |
| Pseudomonas oleovorans ATCC 8062 | 18 | 164 | 207 |
| Saricina lutea ATCC 272 | 0 | 158 | 70 |
| Serratia marcescens ATCC 14225 | 76 | 29 | 0 |

Example 2

In 50 ml of medium similar to Example 1 charged in a 500 ml flask, *Bacillus licheniformis* ATCC 21417 was cultured at 30°C for 16 hours. Into the culture solution was poured under sterile conditions 10 ml of an aqueous solution (adjusted pH to 7.0) containing 500 mg of D-OOC. After adjusting pH of the culture solution to 7.0 under sterile conditions, incubation was carried out for further 10 hours. During the incubation, the pH was adjusted under sterile conditions to 7.0 every two hours.

A part of the culture solution was withdrawn to determine quantitatively L-OOC by liquid chromatography using a resin for optical resolution. L-OOC was formed in an amount of 406 mg/dl.

Example 3

*Bacillus licheniformis* ATCC 21417 was inoculated on 50 ml of medium similar to Example 1 charged in a 500 ml flask. After culturing at 30°C for 16 hours, the cells were centrifuged, washed and collected. The cells were added to phosphate buffer (0.1 M at the end; terminal pH, 7.0) containing 1% of L- or D-OOC in a concentration of 5% calculated as viral bacteria, and allowed to stand at 30°C for 18 hours while the reaction proceeded. After completion of the reaction, the amount of D-OOC and L-OOC in the reaction solution was quantitatively determined by liquid chromatography using a resin for optical resolution. The results are shown in Table 2.

# EP 0 187 525 B1

## TABLE 2
### Amount of L- and D-OOC Produced When L- or D-OOC is Used as Substrate

| Amount of OOC Prior to Reaction [mg/dl] | Amount of OOC After Reaction [mg/dl] |
|---|---|
| L-OOC 1000 | L-OOC: 509 <br> D-OOC: 473 |
| D-OOC 1000 | L-OOC: 492 <br> D-OOC: 471 |

## Example 4

In 50 ml of medium similar to Example 1 charged in a 500 ml flask, *Bacillus licheniformis* ATCC 21417 was cultured at 30°C for 16 hours. After adding 5 ml of a 4% sodium alginate solution to 5 ml of a suspension of the cells in physiological saline in a concentration of 20 g/dl and mixing them, the mixture was slowly dropwise added to a 15 g/dl calcium chloride solution to prepare bead-like immobilized cells. The total amount of the immobilized cells were poured into phosphate buffer (0.1 M at the end; terminal pH, 7.0) containing 1% D-OOC followed by reacting at 30°C for 16 hours. As a result, D-OOC was racemized and 432 mg/dl of L-OOC was formed in the reaction solution.

## Example 5

In a 500 ml volume flask was charged 50 ml of medium (pH 7.0) containing 2% of glycerol, 0.5% of yeast extract, 0.5% of peptone, 0.25% of NaCl, 0.2% of DL-OOC and 4.0% of calcium carbonate (separately sterilized) followed by sterilization at 120°C for 15 minutes.

Using a platinum earpick a microorganism shown in Table 3 cultured at 30°C for 24 hours in bouillon-agar medium was inoculated on the medium. After culturing at 30°C for 20 hours, the cells were collected from the culture solution by centrifugation. The cells were washed with an equivalent amount of physiological saline to that of the culture solution. The cells were collected and added to a reaction solution containing 1% of L-OOC, the pH of which had been adjusted to 7 or 8.5 in a 5% concentration calculated as viral cells. The mixture was allowed to stand at 30°C for 48 hours while the reaction proceeded. After completion of the reaction, L-serine was quantitatively determined by bioassay. The results are shown in Table 3.

## TABLE 3

### Amount of L-Serine Accumulated in Various Microorganisms at pH 7.0 or pH 8.0

| Microorganism | Amount of L-Serine Accumulated [mg/dl] | |
|---|---|---|
|  | pH 7.0 | pH 8.5 |
| Alcaligenes faecalis <br> AJ 2541, FERM-BP 940 | 20 | 58 |
| Arthrobacter grobiformis <br> ATCC 8010 | 4 | 10 |
| Bacillus subtilis <br> ATCC 13952 | 10 | 13 |
| Cellulomonas flavigena <br> ATCC 491 | 41 | 31 |
| Corynebacterium hydrocarboclastus <br> FERM-P 1097 | 4 | 14 |
| Flavobacterium aquatile <br> ATCC 3375 | 26 | 23 |
| Jensenia canicruria <br> ATCC 11048 | 18 | 23 |
| Mycobacterium ammoniaphilum <br> ATCC 15354 | 66 | 42 |

9

TABLE 3 (Continued)

| Microorganism | Amount of L-Serine Accumulated [mg/dl] | |
|---|---|---|
| | pH 7.0 | pH 8.5 |
| Micrococcus roseus ATCC 9815 | 124 | 26 |
| Rhodococcus erythropolis ATCC 4277 | 26 | 23 |
| Pseudomonas testosteroni ATCC 17409 | 368 | 132 |
| Pseudomonas acidovorans ATCC 15668 | 328 | 142 |
| Candida zeylanoides IFO 0719 | 28 | 36 |
| Citeromyces materitensis CBS 2764 | 15 | 23 |
| Cryptococcus laurenti IFO 0609 | 15 | 39 |
| Debaryomyces hansenii IFO 0023 | 13 | 29 |
| Endomycopsis ovetensis CBS 2508 | 30 | 33 |
| Geotricum fragrans CBS 15225 | 20 | 26 |
| Hansenula carifornica IFO 0800 | 17 | 26 |
| Kluyveromyces marxianus IFO 0219 | 20 | 33 |
| Nadosonia falvescens IFO 0666 | 15 | 19 |
| Rhodotorula marina IFO 0879 | 20 | 54 |
| Torulopsis famata IFO 0623 | 20 | 28 |
| Trichosporon fermentans IFO 1199 | 24 | 33 |
| Wickerphamia fluorescens IFO 1116 | 28 | 36 |
| Achromobacter viscosus ATCC 12448 | 95 | 121 |
| Aeromonas salmonicida ATCC 14174 | 58 | 0 |
| Agrobacterium radiobacter ATCC 6466 | 32 | 94 |

10

# EP 0 187 525 B1

TABLE 3 (Continued)

| Microorganism | Amount of L-Serine Accumulated [mg/dl] | |
|---|---|---|
| | pH 7.0 | pH 8.5 |
| Azotobacter vinelandii ATCC 9046 | ·5 | 0 |
| Brevibacterium pusillum ATCC 19096 | 36 | 9 |
| Escherichia coli ATCC 13071 | 14 | 2 |
| Klebsiella penumoniae ATCC 8329 | 12 | 26 |
| Kluyvera non-citrophila FERM-P 3150 | 0 | 36 |
| Kurthina zophii ATCC 6900 | 22 | 7 |
| Mycoplana dimorpha ATCC 4279 | 35 | 30 |
| Proteus-rettgeri AJ 2770, FERM-BP 941 | 31 | 4 |
| Salmonella schottmuelleri ATCC 8759 | 51 | 43 |
| Serratia marcescens ATCC 14225 | 57 | 25 |
| Streptomyces humifer FERM-P 2347 | 0 | 31 |
| Vibrio tyrogenes ATCC 7085 | 27 | 52 |
| Xanthomonas canpestris ATCC 7381 | 17 | 20 |
| Pichia membranaefaciens IFO 0460 | 48 | 37 |
| Saccharomyces fermentati IFO 0422 | 63 | 42 |
| Tremella brasiliensis IFO 9289 | 47 | 33 |
| Alternaria cucumerina IFO 7417 | 98 | 12 |
| Curvularia geniculata ATCC 6671 | 195 | 79 |
| Fursarium nivale ATCC 42308 | 117 | 54 |
| Helminthosporium gramineum ATCC 6695 | 183 | 71 |

11

# EP 0 187 525 B1

TABLE 3 (Continued)

| Microorganism | Amount of L-Serine Accumulated [mg/dl] | |
|---|---|---|
| | pH 7.0 | pH 8.5 |
| Phoma destructiva ATCC 24636 | 103 | 86 |
| Sclerotium bataticola ATCC 12265 | 155 | 140 |
| Cochliobolus miyabeanus IFO 5844 | 264 | 71 |
| Mucor circinelloides ATCC 8770 | 113 | 48 |
| Aspergillus repens ATCC 5817 | 192 | 76 |
| Penicillium decumbens ATCC 10436 | 102 | 24 |
| Gromerella cingulata ATCC 11326 | 31 | 0 |
| Septoria glycines ATCC 38699 | 51 | 3 |
| Pseudoplea trifolii IFO 7252 | 77 | 32 |
| Stemphylium astragali IFO 7244 | 8 | 0 |
| Diplodia natalensis ATCC 34643 | 42 | 0 |
| Stachylidium bicolor ATCC 12672 | 70 | 6 |
| Eupenicillium alutaceum ATCC 18542 | 35 | 7 |
| Anixiella reticulata ATCC 34511 | 102 | 35 |
| Arachniotus flavoluteus ATCC 18430 | 84 | 0 |
| Byssochlamys fulva ATCC 10099 | 51 | 16 |
| Coniochaeta tetraspora ATCC 22275 | 36 | 0 |
| Gelasinospora longispora ATCC 18493 | 45 | 3 |
| Gymnoascus umbrinus IFO 8358 | 102 | 25 |
| Microascus cinerus ATCC 16594 | 50 | 8 |

12

# EP 0 187 525 B1

TABLE 3 (Continued)

| Microorganism | Amount of L-Serine Accumulated [mg/dl] | |
|---|---|---|
| | pH 7.0 | pH 8.5 |
| Microthecium retisporum ATCC 22184 | 100 | 23 |
| Sordaria humana ATCC 22796 | 171 | 43 |
| Sporormiella isomera ATCC 24341 | 152 | 14 |
| Toxotricum cancellatum ATCC 15316 | 35 | 0 |
| Melanospora zamiae ATCC 12340 | 81 | 10 |

## Example 6

In 50 ml of medium similar to Example 1 charged in a 500 ml flask, *Pseudomonas acidovorans* ATCC 15668 was cultured at 30°C for 12 hours. Into the culture solution was poured under sterile conditions 10 ml of an aqueous solution (adjusted pH to 7.0) containing 500 mg of DL-OOC. After adjusting the pH of the culture solution to 7.0 under sterile conditions, incubation was carried out for further 10 hours. During incubation, the pH was adjusted under sterile conditions to 7.0 every 2 hours.

A part of the culture solution was withdrawn and appropriately diluted to determine quantitatively L-serine by bioassay. L-serine was formed in an amount of 288.3 mg/dl.

## Example 7

*Pseudomonas testosteroni* ATCC 17409 was inoculated on 50 ml of medium similar to Example 5 charged in a 500 ml flask. After culturing at 30°C for 16 hours, the culture solution was centrifuged, washed and freeze dried. The cells were suspended in 1 liter of an aqueous solution containing 1% of L-OOC and 1% of $KH_2PO_4$. The reaction was performed by allowing to stand at pH of 7.0 at 30°C for 48 hours. After completion of the reaction, the reaction mixture was centrifuged. The supernatant was taken. A part of the supernatant was appropriately diluted and L-serine produced was quantitatively assayed by bioassay.

L-serine accumulated in an amount of 36.42 mg/dl (molar yield, 45%).

Alternatively, after completion of the reaction, the reaction mixture was centrifuged to remove bacteria. After the supernatant was obtained, 5 g of activated charcoal was added. The mixture was heated and filtered to obtain 990 ml of the supernatant. After the supernatant was concentrated under reduced pressure, the pH was adjusted to 3.0 and passed through a column packed with 500 ml of cationic ion exchange resin Dia Ion SK-1B. After washing with 2000 ml of distilled water, elution was performed, with 2N aqueous ammonia to collect serine fractions. The fractions were concentrated under reduced pressure. After the pH of the concentrate was adjusted to 5.7, approximately 2-fold amount of methanol was slowly added thereto at low temperature to precipitate L-serine crystals. The system was allowed to stand for a further day at 10°C. The precipitated crystals were separated by filtration, washed with methanol and dried to obtain 1.2 g of the crystals.

## Example 8

In 50 ml of medium similar to Example 1 charged in a 500 ml flask, *Pseudomonas testosteroni* ATCC 17409 was cultured at 30°C for 16 hours. The cells were suspended in physiological saline in a concentration of 20 g/dl and 5 ml of a 4% sodium alginate solution was added to 5 ml of the suspension. After mixing, the mixture was slowly dropwise added to a 15 g/dl calcium chloride solution to prepare bead-like immobilized cells. The whole amount of the immobilized cells was poured into phosphate buffer (0.1 M at the end; terminal pH, 7.0) containing 1% of L-OOC followed by reacting at 30°C for 16 hours. As a result, 354 mg/dl of L-serine was formed.

## Example 9

*Bacillus licheniformis* ATCC 21417 was inoculated on medium similar to Example 1. After culturing at 30°C for 16 hours, the cells were centrifuged, washed and collected. The cells were added to phosphate buffer (0.1 M at the end; terminal pH, 7.0) containing 1% of D-OOC in a 5% concentration calculated as viral cells. The system was allowed to stand at 30°C for 48 hours for the reaction to proceed. After completion of

13

# EP 0 187 525 B1

the reaction, a 5% concentration as viral cells of *Pseudomonas testosteroni* ATCC 17409 cultured under the same conditions was added to the reaction solution. The mixture was allowed to stand at 30°C for further 48 hours while the reaction proceeded. After completion of the reaction, L-serine was quantitatively determined by bioassay, showing that 359 mg/dl of L-serine was formed. On the other hand, when only viral cells of *Pseudomonas testosteroni* ATCC 17409 was reacted, no formation of L-serine was observed.

## Claims

1. A process for producing L-serine which comprises reacting a microorganism or cell product thereof capable of producing L-serine from L-2-oxo-oxazolidine-4-carboxylic acid or a salt thereof with a starting material comprising L-2-oxo-oxazolidine-4-carboxylic acid or a salt thereof and producing L-serine.

2. A process according to claim 1 which further comprises reacting a microorganism or cell product thereof capable of racemizing 2-oxo-oxazolidine-4-carboxylic acid or a salt thereof with 2-oxo-oxazolidine-4-carboxylic acid or a salt thereof to produce DL-2-oxo-oxazolidone-4-carboxylic acid or a salt thereof from which L-serine is produced.

## Patentansprüche

1. Verfahren zur Herstellung von L-Serin, gemäß dem ein Mikroorganismus oder dessen Zellprodukt mit der Fähigkeit, L-Serin aus L-2-Oxo-oxazolidin-4-carbonsäure oder aus einem Salz davon herzustellen, mit einem Ausgangsmaterial, das L-2-Oxo-oxazolidin-4-carbonsäure oder ein Salz davon enthält, umgesetzt wird und L-Serin gebildet wird.

2. Verfahren nach Anspruch 1, bei dem zusätzlich ein Mikroorganismus oder dessen Zellprodukt mit der Fähigkeit, 2-Oxo-oxazolidin-4-carbonsäure oder ein Salz davon zu racemisieren, mit 2-Oxo-oxazolidin-4-carbonsäure oder einem Salz davon umgesetzt wird, um DL-2-oxazolidin-4-carbonsäure oder ein Salz davon zu produzieren, woraus L-Serin hergestellt wird.

## Revendications

1. Un procédé pour la fabrication de L-sérine qui consiste à faire réagir un micro-organisme ou son produit cellulaire capable de produire la L-sérine à partir de l'acide L-2-oxo-oxazolidine-4-carboxylique ou d'un de ses sels avec un produit de départ comprenant l'acide L-2-oxo-oxazolidine-4-carboxylique ou un de ses sels et à produire la L-sérine.

2. Un procédé selon la revendication 1 qui consiste en outre à faire réagir un micro-organisme ou son produit cellulaire capable de racémiser l'acide 2-oxo-oxazolidine-4-carboxylique ou un de ses sels avec l'acide 2-oxo-oxazolidine-4-carboxylique ou un de ses sels pour produire l'acide 2-oxo-oxazolidine-4-carboxylique ou un de ses sels à partir duquel la L-sérine est produite.